# FASCICULE DE BREVET EUROPEEN

(11) **EP 3 852 851 B1**
(45) Date de publication et mention de la délivrance du brevet: **13.07.2022**
(21) Numéro de dépôt: 19790652.2
(22) Date de dépôt: 17.09.2019
(51) Int. Cl.: A61M 15/08, A61M 11/00, A61M 11/02, A61M 15/00, B05B 11/00, B05B 11/02

(54) **DISPOSITIF DE DISTRIBUTION NASALE DE PRODUIT FLUIDE**
VORRICHTUNG ZUR NASALEN VERABREICHUNG EINES FLÜSSIGPRODUKTS
DEVICE FOR NASAL DELIVERY OF FLUID PRODUCT

(30) Priorité: 19.09.2018 FR 1858448
(43) Date de publication de la demande: 28.07.2021
(73) Titulaire: APTAR France SAS, 27110 Le Neubourg (FR)
(72) Inventeur: CAZZOLA, Charles, 76000 Rouen (FR); NARBONE, Nicolas, 27930 Gravigny (FR)
(74) Mandataire: CAPRI
(86) Numéro de dépôt international: PCT/FR2019/052157
(87) Numéro de publication internationale: WO 2020/058620

(56) Documents cités:
- WO-A2-2006/106208
- WO-A2-2008/086413
- DE-U1- 20 022 559
- DE-U1-202014 008 893
- FR-A1- 2 771 012
- FR-A1- 2 798 068
- GB-A- 2 412 326
- US-A- 5 901 883
- US-A- 5 961 489

## Description

La présente invention concerne un dispositif de distribution nasale de produit fluide.

Les dispositifs de distribution nasale sont bien connus. Ils comportent généralement un réservoir contenant une ou plusieurs doses de produits fluide et une tête de distribution mobile par rapport audit réservoir pour distribuer le produit fluide, notamment via une pompe, une valve doseuse ou un piston coulissant dans ledit réservoir. Lorsque l'utilisateur souhaite utiliser le dispositif, il insère la tête de distribution dans la narine et actionne le dispositif pour distribuer une dose de produit fluide, généralement sous forme de spray.

Un inconvénient avec les dispositifs de l'art antérieur concerne l'efficacité de la dose distribuée dans la narine, en particulier lorsque le produit fluide distribué a pour objectif d'agir sur le cerveau. En effet, seule une partie minime de la dose atteint généralement la zone cible pour ce type de traitement, à savoir la zone olfactive comprenant les éthmoïdes, notamment en raison d'une orientation du dispositif d'administration dans la narine qui est variable d'un patient à un autre. Or, il apparait que cette orientation conditionne la bonne visée de la zone cible, en particulier dans le cas d'un spray fermé utilisé pour obtenir le maximum de déposition dans la zone cible.

Le document WO2018109409 décrit un ensemble de distribution nasale, associant un dispositif de distribution de produit fluide à un organe de positionnement avec plusieurs zones d'appui facial. Cet ensemble est peu discret, relativement encombrant et comporte deux sous-ensembles séparés.

Les documents WO2006106208 et FR2798068 décrivent d'autres dispositifs de l'état de la technique.

La présente invention a pour but de fournir un ensemble de distribution nasale qui ne reproduit pas les inconvénients susmentionnés.

La présente invention a notamment pour but de fournir un dispositif de distribution nasale permettant de maîtriser l'orientation du dispositif dans la narine.

La présente invention a aussi pour but de fournir un dispositif de distribution nasale qui améliore le taux de déposition de produit actif sur la zone olfactive et/ou les éthmoïdes.

La présente invention a également pour but de fournir un dispositif de distribution nasale qui est simple et peu coûteux à fabriquer et à assembler.

La présente a donc pour objet un dispositif de distribution nasale de produit fluide comprenant un corps comportant un manchon creux et une surface supérieure, un organe d'actionnement monté coulissant dans ledit corps pour réaliser l'actionnement du dispositif, le dispositif comportant deux extensions nasales, dont au moins une forme une tête de distribution pourvue d'un orifice de distribution respectif, lesdites extensions nasales s'étendant à partir de ladite surface supérieure, chaque tête de distribution étant reliée en cours d'actionnement à un réservoir respectif disposé dans ledit corps, lesdites deux extensions nasales formant un angle compris entre 30° et 60° par rapport à un axe central longitudinal dudit corps.

Avantageusement, lesdites deux extensions nasales sont parallèles.

Avantageusement, lesdites deux extensions nasales forment un angle compris entre 40° et 50°, notamment environ 45°.

Avantageusement, lesdites deux extensions nasales ont une profondeur d'insertion comprise entre 5 mm et 15 mm, notamment environ 10 mm.

Avantageusement, chaque réservoir contient une dose unique de produit fluide distribuée en un seul actionnement.

Selon une première variante avantageuse, ledit produit fluide est un liquide, chaque réservoir comportant un tube creux contenant un piston, chaque tête de distribution étant reliée à une aiguille respective, fixe dans ledit corps, pour percer ledit piston lors de l'actionnement.

Selon une seconde variante avantageuse, ledit produit fluide est une poudre, chaque réservoir comportant un tube creux comportant d'un côté une entrée d'air et de l'autre côté une sortie de produit, ladite entrée d'air étant pourvue d'un organe de retenue de produit adapté à maintenir la poudre dans ledit réservoir avant l'actionnement et étant reliée à une chasse d'air formée par ledit organe d'actionnement, et ladite sortie de produit étant obturée avant actionnement par un élément de fermeture, tel qu'une bille sphérique, ladite chasse d'air étant actionnée manuellement par l'utilisateur pour créer un écoulement d'air qui traverse ledit réservoir pour emmener la poudre qu'il contient en direction du ou des orifice(s) de distribution.

Avantageusement, le dispositif comporte un système d'ouverture mécanique, solidaire de la chasse d'air, adapté à coopérer avec ledit élément de fermeture pour l'expulser mécaniquement de sa position d'obturation lors de l'actionnement du dispositif.

Selon un premier mode de réalisation avantageux, les deux extensions nasales forment chacune une tête de distribution, pourvue chacune d'un orifice de distribution respectif.

Avantageusement, le corps contient deux réservoirs, dont chacun est relié à une tête de distribution respective lors de l'actionnement, pour distribuer du produit fluide simultanément dans les deux narines.

Selon un second mode de réalisation avantageux, l'une des extensions nasales forme une tête de distribution pourvue d'un orifice de distribution, pour distribuer du produit fluide dans l'une des deux narines, et l'autre extension nasale forme un guide, pour coopérer avec l'autre narine.

Avantageusement, une partie centrale de ladite surface supérieure dudit corps, située entre lesdites deux extensions nasales, vient s'appuyer lors de l'actionnement sur la columelle du nez.

Avantageusement, ladite partie centrale comporte un profil légèrement concave pour s'adapter à la columelle du nez.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective de face d'un dispositif de distribution nasale selon un premier mode de réalisation avantageux,
- la figure 2 est une vue schématique en perspective de côté du dispositif de la figure 1,
- la figure 3 est une vue schématique en perspective de dessus du dispositif des figures 1 et 2,
- la figure 4 est une vue schématique en perspective d'un dispositif de distribution nasale selon un second mode de réalisation avantageux,
- la figure 5 est une vue schématique illustrant une manière d'utiliser le dispositif des figures 1 à 3,
- la figure 6 est une vue schématique en section transversale d'un dispositif de distribution nasale selon les figures 1 à 3, dans une première variante de réalisation avantageuse, avant utilisation,
- la figure 7 est une vue similaire à celle de la figure 6, après utilisation,
- la figure 8 est une vue schématique en section transversale d'un dispositif de distribution nasale selon les figures 1 à 3, dans une seconde variante de réalisation avantageuse, avant utilisation,
- la figure 9 est une vue similaire à celle de la figure 8, après utilisation,
- la figure 10 est une vue schématique en section transversale d'un dispositif de distribution nasale selon la figure 4, dans une première variante de réalisation avantageuse, avant utilisation, et
- la figure 11 est une vue schématique en section transversale d'un dispositif de distribution nasale selon la figure 4, dans une seconde variante de réalisation avantageuse, avant utilisation.

Le dispositif de distribution de produit fluide représenté sur les figures comporte un corps 10 sur lequel sont fixés deux extensions nasales 20, 30, dont au moins une est une tête de distribution nasale. Ces extensions nasales 20,30 s'étendent à partir d'une surface supérieure 15 dudit corps, et sont destinées à pénétrer chacune dans une narine respective lors de l'utilisation du dispositif. De préférences, les extensions axiales 20, 30 sont relativement souples pour éviter tout risque de blessure lors de leur insertion dans les narines.

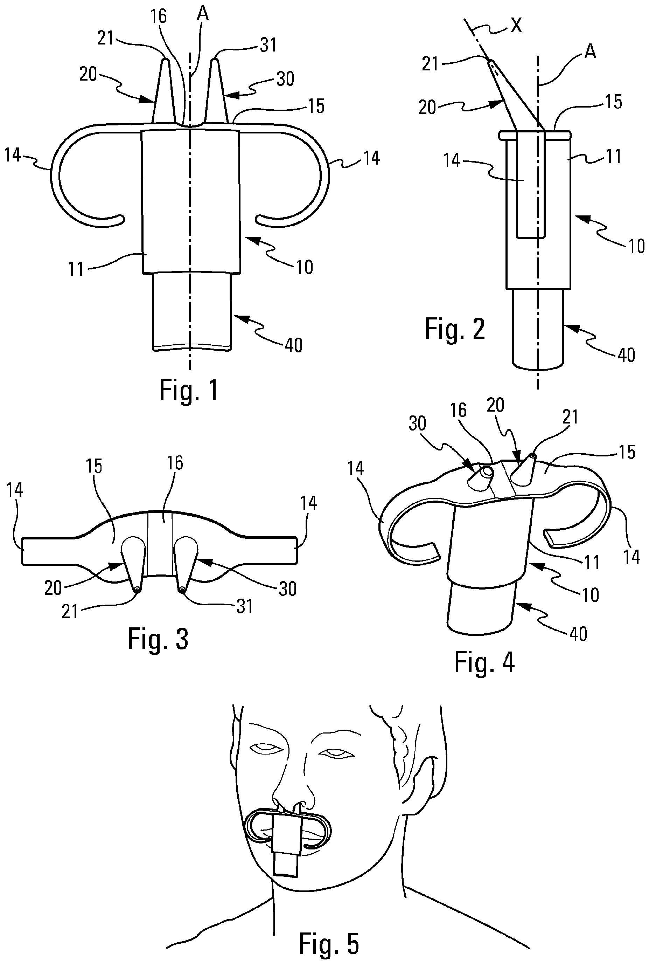

La partie centrale 16 de ladite surface supérieure 15 du corps 10, qui est située entre les deux extensions axiales 20, 30 vient s'appuyer lors de l'actionnement sur la columelle du nez. Avantageusement, cette partie centrale 16 peut avoir un profil légèrement concave ou incurvé pour s'adapter à ladite columelle, comme visible notamment sur la figure 4.

Dans un premier mode de réalisation avantageux, représenté sur les figures 1 à 3 et 5 à 9, les deux extensions nasales 20, 30 forment chacune une tête de distribution nasale, respectivement pourvue d'un orifice de distribution 21, 31. Dans ce premier mode de réalisation, dont deux variantes seront décrites plus en détail ci-après, l'actionnement du dispositif provoque la distribution simultanée de produit fluide dans les deux narines.

Dans un second mode de réalisation avantageux, représenté sur les figures 4, 10 et 11, l'une des extensions nasales 20 forme une tête de distribution nasale, pourvue d'un orifice de distribution 21, et l'autre extension nasale 30 forme un guide. Dans ce second mode de réalisation, dont deux variantes seront décrites plus en détail ci-après, l'actionnement du dispositif provoque la distribution de produit fluide dans une seule narine.

Les deux extensions nasales 20,30 sont inclinées, c'est-à-dire que l'axe X le long duquel s'étendent les deux extensions axiales 20, 30 forme un angle d'insertion par rapport à l'axe longitudinal A du corps 10, qui est un axe vertical dans l'orientation des figures 1, 2 et 6 à 11. De préférence, les deux extensions nasales 20, 30 sont parallèles.

Le dispositif selon l'invention permet d'orienter la ou les tête(s) de distribution dans chaque narine d'une manière sensiblement prédéterminée, et donc de cibler les zones de distribution du produit fluide dans la ou les narines.

Ainsi, l'angle d'insertion de chaque extension nasale 20, 30 dans la narine respective est compris entre 30° et 60°, avantageusement entre 40° et 50°, de préférence environ 45°.

Par ailleurs, la profondeur d'insertion P de chaque tête de distribution dans la narine est prédéterminée par les dimensions des extensions axiales 20, 30 par rapport à la surface supérieure 15 du corps 10, et en particulier par rapport à la partie centrale 16 qui s'appuie sur la columelle du nez. Cette profondeur d'insertion est comprise avantageusement entre 5 mm et 15 mm, de préférence environ 10 mm.

Le corps 10 comporte un manchon creux 11 qui reçoit un ou deux réservoirs 50, comme cela sera décrit ci-après.

Avantageusement, le corps 10 comporte également deux arceaux latéraux externes 14 adaptés à recevoir les doigts de l'utilisateur, en particulier l'index d'une part et le majeur d'autre part. En variante, l'utilisateur peut appuyer avec ses doigts sur la surface supérieure 15, par exemple radialement à l'extérieur des deux extensions nasales 20, 30.

Un organe d'actionnement 40 est monté coulissant dans le corps 10, pour actionner le dispositif. Typiquement, l'utilisateur place son pouce sur le fond dudit organe d'actionnement 40 pour réaliser l'actionnement.

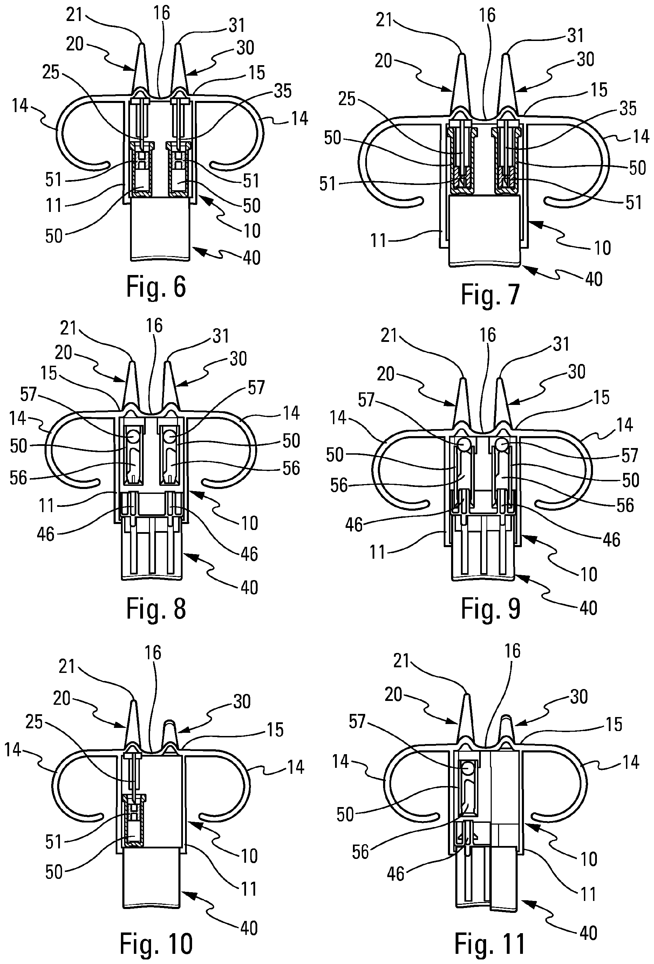

Le dispositif peut être utilisé pour distribuer un produit liquide, comme représentés sur les figures 6, 7 et 10, ou un produit pulvérulent, comme représenté sur les figures 8, 9 et 11.

Selon un premier mode de réalisation avantageux, le dispositif réalise la distribution simultanée de produit fluide dans les deux narines.

Pour ce faire, les deux extensions nasales 20, 30 forment chacune une tête de distribution nasale, chacune étant reliée à un réservoir respectif 50.

De préférence, chaque réservoir 50 contient une dose unique de produit fluide qui sera distribuée en un seul actionnement. Le dispositif est alors appelé unidose, car la totalité du produit fluide est distribué en un seul actionnement, même si une dose est distribuée simultanément dans chaque narine. Eventuellement, on pourrait appliquer la présente invention à des dispositifs multidoses, par exemple des bidoses.

Les figures 6 et 7 illustrent une première variante du premier mode de réalisation, dans lequel le produit fluide est un liquide. Dans cette variante, chaque réservoir 50 est fixé dans l'organe d'actionnement 40 et donc déplaçable axialement par rapport au corps 10 lors de l'actionnement.

Chaque réservoir 50 est de préférence formé d'un tube creux borgne comportant une seule ouverture axiale obturée par un piston 51 formant bouchon étanche avant actionnement. Chaque tête de distribution est reliée à une aiguille 25, 35, fixe par rapport au corps 10, qui lors de l'actionnement, va percer le piston 51 respectif et permettre la distribution simultanée du produit fluide contenu dans chaque réservoir 50 à travers les orifices de distribution 21, 31 au fur et à mesure que chaque piston 51 coulisse dans le réservoir respectif 50.

Les figures 8 et 9 illustrent une seconde variante du premier mode de réalisation, dans lequel le produit fluide est une poudre. Cette seconde variante utilise avantageusement des moyens similaires à ceux décrits dans le document WO2015001281.

Dans cette seconde variante, chaque réservoir 50 est fixé dans le corps 10 et donc non déplaçable axialement par rapport au corps 10 lors de l'actionnement.

Chaque réservoir 50 est formé d'un tube creux comportant d'un côté une entrée d'air et de l'autre côté une sortie de produit. L'entrée d'air de chaque réservoir est reliée à une chasse d'air et la sortie de produit de chaque réservoir est reliée à une tête de distribution respective. La sortie de produit est obturée avant actionnement par un élément de fermeture 57, de préférence une bille sphérique, qui est emmanché à force dans ladite sortie de produit. L'entrée d'air est pourvue d'un organe de retenue de produit qui est adapté à maintenir la poudre dans le réservoir avant l'actionnement du dispositif. La chasse d'air est formée par l'organe d'actionnement 40 et est actionnée manuellement par l'utilisateur pour créer un écoulement d'air qui va traverser chaque réservoir 50 pour emmener le produit qu'il contient en direction de l'orifice de distribution 21, 31 respectif.

Le dispositif comporte un système d'ouverture mécanique 46, 56, qui est solidaire de la chasse d'air, c'est à dire qu'il est actionné simultanément à l'actionnement de ladite chasse d'air, et qui est adapté à coopérer avec ledit élément de fermeture 57 pour l'expulser mécaniquement de sa position d'obturation lors de l'actionnement du dispositif. Le système d'ouverture mécanique peut comporter un ensemble de tiges 46, 56, dont une première partie de tige 46 est solidaire de l'organe d'actionnement 40 formant la chasse d'air 20, et une seconde partie de tige 56, disposée dans le réservoir 50, est poussée par ladite première partie de tige 46 lorsque le dispositif est actionné. L'ensemble de tiges 46, 56 va en fin de course d'actionnement coopérer avec l'élément de fermeture 57 pour l'expulser mécaniquement de sa position d'obturation.

L'organe de retenue de produit peut être avantageusement réalisé monobloc avec la seconde partie de tige 56. Ainsi, l'organe de retenue de produit peut être réalisé de manière étanche au produit et étanche à l'air, avant l'actionnement du dispositif, la pression d'air créée par la chasse d'air ne pénétrant à l'intérieur du réservoir 50 qu'au moment où ledit organe de retenu est déplacé en étant poussé par la première partie de tige 46.

La chasse d'air, non représentée en détails, comporte un piston, par exemple formé sur l'organe d'actionnement 40, coulissant dans une chambre d'air, par exemple formée dans le manchon 11 du corps 10, le piston étant actionné manuellement par l'utilisateur lorsqu'il déplace l'organe d'actionnement 40 par rapport au corps 10.

Selon un second mode de réalisation avantageux, le dispositif réalise la distribution de produit fluide dans une seule narine.

Pour ce faire, seule l'une des extensions nasales 20, 30 forme une tête de distribution nasale, reliée à un réservoir 50. L'autre extension nasale forme un guide, destiné à pénétrer dans la seconde narine dans des buts de guidage et d'orientation du dispositif. De préférence, la tête de distribution et le guide sont parallèles, comme visible sur la figure 4.

La figure 10 illustre une première variante de ce second mode de réalisation, dans lequel le produit fluide est un liquide. Cette variante est similaire à celle des figures 6 et 7 décrite précédemment.

La figure 11 illustre une seconde variante de ce second mode de réalisation, dans lequel le produit fluide est une poudre. Cette variante est similaire à celle des figures 8 et 9 décrite précédemment.

La présente invention fournit ainsi un dispositif à la fois discret, peu encombrant, aisément transportable, d'utilisation intuitive, avec une orientation et une profondeur d'insertion appropriées et conformes à la morphologie de l'utilisateur.

La présente invention est particulièrement adaptée dans le cas de traitements du système nerveux central tels que ceux visant les maladies neuro dégénératives, type Parkinson ou Alzheimer, nécessitant de faire transiter le médicament au travers de la barrière nez-cerveau. L'atteinte de la zone cible, en particulier les ethmoïdes, par la cavité nasale est un moyen simplifié et non invasif d'administrer un traitement dans le cerveau. D'autres applications sont envisageables, telles que les traitements de l'autisme, de différentes formes d'épilepsie, des migraines, des maladies neurologiques, du trouble de déficit de l'attention avec ou sans hyperactivité (TDAH), de la démence, de la sclérose en plaques, des maladies cérébrovasculaires, des tumeurs cérébrales. Les molécules suivantes peuvent être concernées : insuline, oxytocine, dopamine, lévodopa, thérapie par cellules souches (tumeurs cérébrales), mésylate de rasagiline, ropinirole, amantadine, sumatriptan, huperzine A, curcumin, geniposide, rivastigmine, tartrate de rivastigmine, Doxepin, Agomelatine, hydrochlorure de fluoxetine, hydrochlorure de Tramadol, hydrochlorure de Nortriptyline, venlafaxine, paliperidone. Pour les traitements d'Alzheimer : donépézil, galantamine, rivastigmine, mémantine et combinaison de donépézil et de mémantine.

La présente invention a été décrite en référence à plusieurs modes et variantes de réalisation avantageux, mais il est entendu qu'un homme du métier peut y apporter toutes modifications, sans sortir du cadre de la présente invention tel que défini par les revendications annexées.

## Revendications

1. Dispositif de distribution nasale de produit fluide comprenant un corps (10) comportant un manchon creux (11) et une surface supérieure (15), un organe d'actionnement (40) monté coulissant dans ledit corps (10) pour réaliser l'actionnement du dispositif, **caractérisé en ce que** le dispositif comporte deux extensions nasales (20, 30), dont au moins une forme une tête de distribution pourvue d'un orifice de distribution respectif (21, 31), lesdites extensions nasales s'étendant à partir de ladite surface supérieure (15), chaque tête de distribution étant reliée en cours d'actionnement à un réservoir respectif (50) disposé dans ledit corps (10), lesdites deux extensions nasales (20, 30) formant un angle compris entre 30° et 60° par rapport à un axe central longitudinal (A) dudit corps (10).

2. Dispositif selon la revendication 1, dans lequel lesdites deux extensions nasales (20, 30) sont parallèles.

3. Dispositif selon la revendication 1 ou la revendication 2, dans lequel lesdites deux extensions nasales (20, 30) forment un angle compris entre 40° et 50°, notamment environ 45°.

4. Dispositif selon l'une quelconque des revendications précédentes, dans lequel lesdites deux extensions nasales (20, 30) ont une profondeur d'insertion comprise entre 5 mm et 15 mm, notamment environ 10 mm.

5. Dispositif selon l'une quelconque des revendications précédentes, dans lequel chaque réservoir (50) contient une dose unique de produit fluide distribuée en un seul actionnement.

6. Dispositif selon l'une quelconque des revendications précédentes, dans lequel ledit produit fluide est un liquide, chaque réservoir (50) comportant un tube creux contenant un piston (51), chaque tête de distribution étant reliée à une aiguille (25, 35) respective, fixe dans ledit corps (10), pour percer ledit piston (51) lors de l'actionnement.

7. Dispositif selon l'une quelconque des revendications 1 à 5, dans lequel ledit produit fluide est une poudre, chaque réservoir (50) comportant un tube creux comportant d'un côté une entrée d'air et de l'autre côté une sortie de produit, ladite entrée d'air étant pourvue d'un organe de retenue de produit adapté à maintenir la poudre dans ledit réservoir (50) avant l'actionnement et étant reliée à une chasse d'air formée par ledit organe d'actionnement (40), et ladite sortie de produit étant obturée avant actionnement par un élément de fermeture (57), tel qu'une bille sphérique, ladite chasse d'air étant actionnée manuellement par l'utilisateur pour créer un écoulement d'air qui traverse ledit réservoir (50) pour emmener la poudre qu'il contient en direction du ou des orifice(s) de distribution (21, 31).

8. Dispositif selon la revendication 7, comportant un système d'ouverture mécanique (46, 56), solidaire de la chasse d'air, adapté à coopérer avec ledit élément de fermeture (57) pour l'expulser mécaniquement de sa position d'obturation lors de l'actionnement du dispositif.

9. Dispositif selon l'une quelconque des revendications précédentes, dans lequel les deux extensions nasales (20, 30) forment chacune une tête de distribution, pourvue chacune d'un orifice de distribution respectif (21, 31).

10. Dispositif selon la revendication 9, dans lequel le corps (10) contient deux réservoirs (50), dont chacun est relié à une tête de distribution respective lors de l'actionnement, pour distribuer du produit fluide simultanément dans les deux narines.

11. Dispositif selon l'une quelconque des revendications 1 à 8, dans lequel l'une des extensions nasales (20) forme une tête de distribution pourvue d'un orifice de distribution (21), pour distribuer du produit fluide dans l'une des deux narines, et l'autre extension nasale (30) forme un guide, pour coopérer avec l'autre narine.

12. Dispositif selon l'une quelconque des revendications précédentes, dans lequel une partie centrale (16) de ladite surface supérieure (15) dudit corps (10), située entre lesdites deux extensions nasales (20, 30), vient s'appuyer lors de l'actionnement sur la columelle du nez.

13. Dispositif selon la revendication 12, dans lequel ladite partie centrale (16) comporte un profil légèrement concave pour s'adapter à la columelle du nez.

## Patentansprüche

1. Vorrichtung zur nasalen Verabreichung eines Flüssigprodukts, umfassend einen Körper (10) mit einer hohlen Hülse (11) und einer oberen Fläche (15), wobei ein Betätigungsorgan (40) gleitend in dem Körper (10) montiert ist, um die Betätigung der Vorrichtung zu ermöglichen, **dadurch gekennzeichnet, dass** die Vorrichtung zwei Nasenfortsätze (20, 30) umfasst, von denen mindestens einer einen Abgabekopf bildet, der mit einer jeweiligen Abgabeöffnung (21, 31) versehen ist, wobei sich die Nasenfortsätze von der oberen Fläche (15) aus erstrecken, wobei jeder Abgabekopf bei der Betätigung mit einem jeweiligen in dem Körper (10) angeordneten Vorratsbehälter (50) verbunden ist, wobei die beiden Nasenfortsätze (20, 30) bezüglich einer Längsmittelachse (A) des Körpers (10) einen Winkel zwischen 30° und 60° bilden.

2. Vorrichtung nach Anspruch 1, bei der die beiden Nasenfortsätze (20, 30) parallel verlaufen.

3. Vorrichtung nach Anspruch 1 oder Anspruch 2, bei der die beiden Nasenfortsätze (20, 30) einen Winkel zwischen 40° und 50°, insbesondere etwa 45°, bilden.

4. Vorrichtung nach einem der vorhergehenden Ansprüche, bei der bei den beiden Nasenfortsätzen (20, 30) eine Einführtiefe zwischen 5 mm und 15 mm, insbesondere etwa 10 mm, vorgesehen ist.

5. Vorrichtung nach einem der vorhergehenden Ansprüche, bei der jeder Vorratsbehälter (50) eine Einzeldosis eines Flüssigprodukts enthält, die mit nur einer Betätigung abgegeben wird.

6. Vorrichtung nach einem der vorhergehenden Ansprüche, bei der es sich bei dem Flüssigprodukt um eine Flüssigkeit handelt, wobei jeder Vorratsbehälter (50) ein hohles Rohr mit einem darin enthaltenen Kolben (51) aufweist, wobei jeder Abgabekopf mit einer jeweiligen Nadel (25, 35) verbunden ist, die in dem Körper (10) angebracht ist, um den Kolben (51) bei Betätigung zu durchstechen.

7. Vorrichtung nach einem der Ansprüche 1 bis 5, bei der es sich bei dem Flüssigprodukt um ein Pulver handelt, wobei jeder Vorratsbehälter (50) ein hohles Rohr umfasst, das auf einer Seite einen Lufteinlass und auf der anderen Seite einen Produktauslass aufweist, wobei der Lufteinlass mit einem Produktrückhalteorgan versehen ist, das geeignet ist, um das Pulver vor der Betätigung in dem Behälter (50) zu halten, und mit einer von dem Betätigungsorgan (40) gebildeten Luftausstoßvorrichtung verbunden ist, und der Produktauslass vor der Betätigung durch ein Verschlusselement (57), beispielsweise eine Kugel, verschlossen ist, wobei die Luftausstoßvorrichtung vom Benutzer manuell betätigt wird, um dadurch einen Luftstrom zu erzeugen, der den Behälter (50) durchströmt und dabei das darin enthaltene Pulver in Richtung der Abgabeöffnung(en) (21, 31) befördert.

8. Vorrichtung nach Anspruch 7, umfassend ein mechanisches Öffnungssystem (46, 56), das fest mit der Luftausstoßvorrichtung verbunden ist und geeignet ist, um mit dem Verschlusselement (57) zusammenzuwirken, um es bei der Betätigung der Vorrichtung mechanisch aus seiner Verschlussposition heraus zu drücken.

9. Vorrichtung nach einem der vorhergehenden Ansprüche, bei der jeder der beiden Nasenfortsätze (20, 30) jeweils einen Abgabekopf bildet, der jeweils mit einer entsprechenden Abgabeöffnung (21, 31) versehen ist.

10. Vorrichtung nach Anspruch 9, bei der der Körper (10) zwei Vorratsbehälter (50) enthält, von denen jeder bei Betätigung mit einem jeweiligen Abgabekopf verbunden wird, so dass die Abgabe von Flüssigprodukt in beide Nasenlöcher gleichzeitig erfolgt.

11. Vorrichtung nach einem der Ansprüche 1 bis 8, bei der einer der Nasenfortsätze (20) einen mit einer Abgabeöffnung (21) versehenen Abgabekopf zur Abgabe von Flüssigprodukt in eines der beiden Nasenlöcher bildet und der andere Nasenfortsatz (30) eine mit dem anderen Nasenloch zusammenwirkende Führung bildet.

12. Vorrichtung nach einem der vorhergehenden Ansprüche, bei der ein mittlerer Teil (16) der oberen Fläche (15) des Körpers (10), der sich zwischen den beiden Nasenfortsätzen (20, 30) befindet, bei der Betätigung am Nasensteg zur Anlage kommt.

13. Vorrichtung nach Anspruch 12, bei der der mittlere Teil (16) ein leicht konkaves Profil aufweist, um sich dem Nasensteg anzupassen.

## Claims

1. A nasal fluid dispenser device comprising a body (10) comprising a hollow sleeve (11) and a top surface (15), and an actuator member (40) that is mounted to slide in said body (10) so as to actuate the device, the device being **characterized in that** it further comprises two nasal extensions (20, 30), at least one of which forms a dispenser head provided with a respective dispenser orifice (21, 31), said nasal extensions extending from said top surface (15), each dispenser head being connected, during actuation, to a respective reservoir (50) that is arranged in said body (10), said two nasal extensions (20, 30) forming an angle lying in the range 30° to 60° relative to a longitudinal central axis (A) of said body (10).

2. A device according to claim 1, wherein said two nasal extensions (20, 30) are parallel.

3. A device according to claim 1 or claim 2, wherein said two nasal extensions (20, 30) form an angle lying in the range 40° to 50°, in particular about 45°.

4. A device according to any preceding claim, wherein said two nasal extensions (20, 30) have an insertion depth lying in the range 5 mm to 15 mm, in particular about 10 mm.

5. A device according to any preceding claim, wherein each reservoir (50) contains a single dose of fluid for dispensing in a single actuation.

6. A device according to any preceding claim, wherein said fluid is a liquid, each reservoir (50) comprising a hollow tube containing a piston (51), each dispenser head being connected to a respective needle (25, 35), stationary in said body (10), for piercing said piston (51) during actuation.

7. A device according to any one of claims 1 to 5, wherein said fluid is a powder, each reservoir (50) comprising a hollow tube including an air inlet at one end and a fluid outlet at the other end, said air inlet being provided with a fluid retainer member that is adapted to retain the powder in said reservoir (50) before actuation and being connected to an air expeller that is formed by said actuator member (40), and said fluid outlet being closed before actuation by a closure element (57), such as a spherical ball, said air expeller being actuated manually by the user so as to create a flow of air that passes through said reservoir (50) so as to deliver the powder contained therein towards the dispenser orifice(s) (21, 31).

8. A device according to claim 7, including a mechanical opening system (46, 56) that is secured to the air expeller and that is adapted to co-operate with said closure element (57) so as to expel it mechanically from its closed position while the device is being actuated.

9. A device according to any preceding claim, wherein each of the two nasal extensions (20, 30) forms a dispenser head, each provided with a respective dispenser orifice (21, 31).

10. A device according to claim 9, wherein the body (10) contains two reservoirs (50), each of which is connected to a respective dispenser head during actuation, for dispensing fluid simultaneously into both nostrils.

11. A device according to any one of claims 1 to 8, wherein one of the nasal extensions (20) forms a dispenser head provided with a dispenser orifice (21) for dispensing fluid into one of the two nostrils, and the other nasal extension (30) forms a guide for co-operating with the other nostril.

12. A device according to any preceding claim, wherein a central portion (16) of said top surface (15) of said body (10), situated between said two nasal extensions (20, 30), comes to bear against the septum of the nose during actuation.

13. A device according to claim 12, wherein said central portion (16) has a profile that is slightly concave so as to adapt to the septum of the nose.
